# EUROPEAN PATENT SPECIFICATION

(11) **EP 0 441 886 B1**
(45) Date of publication and mention of the grant of the patent: **21.08.1996**
(21) Application number: 89912976.1
(22) Date of filing: 03.11.1989
(51) Int. Cl.: C12N 15/16, C12N 15/82, C07K 14/00, A61K 38/27

(54) **EXPRESSION AND PROCESSING OF ACETYLATED FGF'S IN YEAST**
EXPRESSION UND PROZESSIERUNG VON ACETYLIERTEN FGFs IN HEFEN
EXPRESSION ET TRAITEMENT DE FGF (FACTEURS DE CROISSANCE DE FIBROBLASTE) ACETYLES DANS DE LA LEVURE

(30) Priority: 04.11.1988 US 267408
(43) Date of publication of application: 21.08.1991
(73) Proprietor: CHIRON CORPORATION, Emeryville, California 94608 (US)
(72) Inventor: BARR, Philip, J., Oakland, CA 94661 (US)
(74) Representative: Hallybone, Huw George
(86) International application number: US8904821
(87) International publication number: WO9005184

(56) References cited:
- EP-A- 0 237 966
- EP-A- 0 259 953
- EP-A- 0 275 204
- WO-A-87/01728
- WO-A-87/05332
- JOURNAL OF BIOLOGICAL CHEMISTRY, vol. 263, no. 31, 1988; P.J. BARR et al., pp. 16471-16478
- BIOCHEMICAL & BIOPHYSICAL RESEARCH COMMUNICATIONS, vol. 135, 1986; pp. 541-548

## Description

### FIELD OF THE INVENTION

The present invention relates generally to the production of human fibroblast growth factors (FGF's) utilizing recombinant DNA technology, and more particularly, to the expression of authentic FGF's in yeast cells.

### BACKGROUND OF THE INVENTION

FGF was first identified as a mitogenic pituitary hormone in the 1970's (Gospodarowicz, D. 1974 Nature 249:123-127). Thereafter, factors present in primarily brain and pituitary tissues were shown capable of stimulating the growth of a number of different cell types, including endothelial cells, fibroblast cells, retinal cells, and others. Only recently, with the cloning of genes encoding the various growth factors, has it become clear that most of the activities reside in two microheterogeneous proteins, basic and acidic FGF's (see, Gospodarowicz, D., (1986) Mol. and Cell. Endocrin. 46:187-204, which is incorporated herein by reference).

Basic and acidic FGF's have now been isolated from many diverse sources and by a variety of purification schemes. Although the production of cDNA and genomic clones encoding FGF precursors have demonstrated the common identity of the various growth factors, new issues have been raised concerning their biology. For example, the cDNA clones of the FGF's did not include classical signal sequences generally associated with secreted proteins. Also, various research groups have reported different NH2-terminus amino acids, as well as varying lengths of the overall FGF proteins.

In order to accurately decipher the different biological characteristics, if any, of the microheterogeneous forms of FGF, it is necessary to establish recombinant expression systems for the production of the various FGF forms. The expression systems should be capable of post-translational modification in the native fashion, including amino-terminal acetylation. Ideally, the systems will also provide for high expression levels of the proteins in forms that can be readily purified. all in an economical manner. The present invention fulfills these and other needs.

### SUMMARY OF THE INVENTION

The present invention provides compositions and methods for the production of human basic and acidic FGF's in yeast, which are typically substantially amino-terminal acetylated. These authentically amino-terminal processed proteins are expressed in the yeast intracellularly at high levels (without secretion) and readily purified to substantial homogeneity. For human basic FGF polypeptides, a specific activity of at least about 9.3 x 105 units/mg was obtained; and for human acidic FGF polypeptides, a specific activity of at least about 0.61 x 105 units/mg was obtained.

Methods of the present invention for producing the authentic post-transnational modified forms of human FGF's comprise the steps of:
a) transforming yeast with an expression plasmid comprising a gene encoding an FGF of about 155 amino acids including N-terminal methionine, wherein the gene is under transcriptional control of a promoter functional in yeast;
b) culturing the transformed yeast under conditions suitable for expression, substantially without secretion, of the FGF gene, wherein N-terminus acetylated FGF polypeptides are produced; and
c) separating the FGF polypeptides from the yeast culture.

In this manner, typically between 30% and 100% of the FGF polypeptides are amino-terminal acetylated. The FGF polypeptides are frequently microheterogeneous, containing from about two to about five modified forms. Each of these polypeptides may be purified to homogeneity by, e.g., a heparin affinity column and/or an HPLC column.

The DNA constructs of the present invention are those capable of directing the intracellular expression in yeast of a final processed human basic or acidic FGF (1-154), and deletions thereof. The constructs can also comprise a yeast transcriptional promoter DNA region upstream from an initiation codon, which is fused to a gene encoding the desired FGF. The gene is typically followed downstream by a transcription terminator. Generally. the construct is not capable of directing the secretion of polypeptides from the transformed yeast cell host.

The compositions containing the acetylated human FGF's of the present invention typically have essentially all the primary structural conformation and one or more of the naturally associated biological properties of native FGF's. These compositions will be substantially free from bacterial or other mammalian proteins. and will typically not include an initial methionine amino acid residue.

### BRIEF DESCRIPTION OF THE FIGURES

Figure 1 shows the amino acid sequence and corresponding preferred synthetic nucleotide sequence for human basic FGF (1-154).

Figure 2 shows the amino acid sequence of human acidic FGF (1-154).

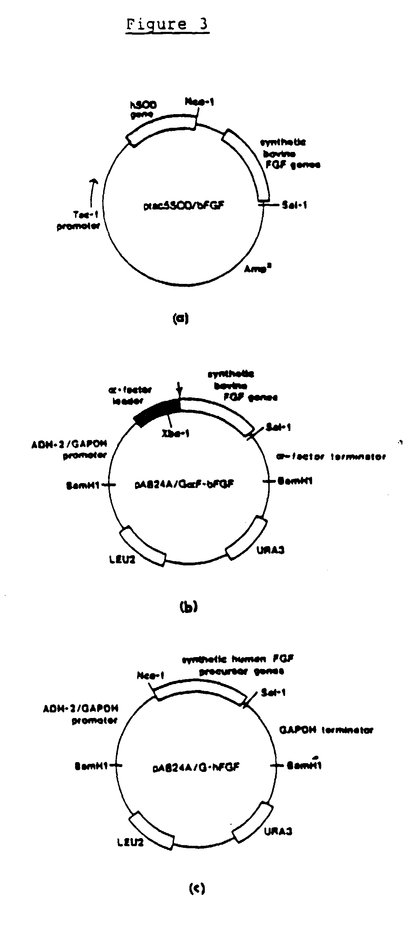

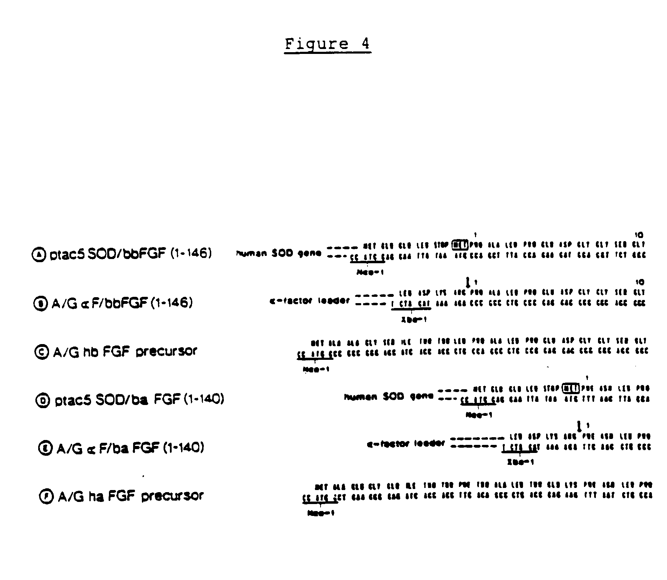

Figure 3 is a schematic representation of plasmid constructions for the expression of bovine and human FGF's. (a) ptac5SOD/bFGF, derived from ptac5SOD, allows the transcription of hSOD, together with bovine acidic FGF (1-140) and bovine basic FGF (1-146) RNA's as dicistronic messages. (b) pAB24a/GaF-baFGF (1-140) and pAB24A/GaF-bbFGF (1-146) yeast secretion plasmids for the expression and processing of a-factor leader bFGF fusion proteins in S. cerevisiae. Transcription is driven by the glucose regulatable ADH-2/GAPDH promoter. (c) pAB24 A/G-haFGF and pAB24 a/G-hbFGF plasmids for the glucose regulatable intracellular expression of human FGF precursors. Again, transcription is driven by the ADA-2/GAPDH promoter. The parent yeast vector pAB24 contains LEU2 and URA3 genes for selection of transformants under leucine or uracil deficient conditions. Detailed DNA and encoded amino acid sequences around the promoter and secretion signal-FGF fusions are shown in Figure 4.

Figure 4 depicts synthetic DNA and encoded amino acid junction sequences in basic FGF (A-C). and acidic FGF (D-F) constructions for expression in E. coli (A.D), secretion from S, cerevisiae (B,E), and for intracellular expression in S. cerevisiae (C,F).

Figure 5 shows SDS Gel analysis of "short" rhbFGF expression. Extracts were made by glass bead lysis of yeast cells, and subjected to 1 hour at 50,000 rpm in an ultracentrifuge. Aliquots of these clarified extracts were analyzed by SDS gel electrophoresis. The rhbFGF's were purified by chromatography on a Heparin 5-PW HPLC column, and portions of the purified protein also electrophoresed.

Figure 6 shows reverse phase HPLC analysis of "short" rhbFGF's 9-154 and 10-154. A portion of the purified rhbFGF's were analyzed by chromatography on a Vydac C-4 reverse phase column at room temperature.

Figure 7 shows treatment of rhbFGF with alakaline phosphatase. Aliquots of gel-purified 17.5 kDa and 19.5 kDa rhbFGF were incubated for 2 hours at 37°C in the presence or absence of calf intestinal alkaline phosphatase, mixed with sample buffer and electrophoresed on a 15% SDS gel.

Figure 8 shows reverse phase HPLC of rhbFGF at elevated temperature. Parts A-C, aliquots of rhbFGF (Lot Hb16) were analyzed by chromatography on a Vydac C-4 column at room temperature (Part A), and at 50°C (Parts B and C). Part C shows a more shallow gradient than A and B. In Part D, a sample of rhbFGF (Lot Hb19) was analyzed at 50°C on a Polymer Labs PLRP-S, 300 Angstrom column.

### DESCRIPTION OF THE SPECIFIC EMBODIMENTS

Methods and compositions are provided for the efficient expression of authentic FGF polypeptides, in particular, acidic and basic FGF's that are acetylated at the amino-terminus during intracellular yeast expression. The methods employ DNA sequences encoding the amino acid sequence of the desired FGF, in conjunction with a translational initiation region optimized for expression in yeast cells. The genes are inserted into a vector capable of intracellular expression (i.e., substantially in the absence of secretion), so the FGF polypeptides are harvested only after the cells have been lysed. Thereafter, the FGF's may be purified for use in a variety of ways, including enhancing in vitro growth of susceptible cells, and as therapeutic agents.

The compositions of the present invention will typically comprise human FGF's that are acetylated. As used herein, acetylation refers to an acetyl group addition at the ammo-terminus of the proteins, such as disclosed in United States Patent 5,066,591 the basic application of which was filed November 5, 1984.

Acetylation is desirable for a number of reasons, most notably to provide a product having the appropriate native structure and confirmation. Thus, for use in pharmaceutical applications, the acetylated FGF's will substantially reduce or eliminate immunogenicity when administered to a host. Further acetylated polypeptides are thought to be more stable and resistant to degradation when utilized in vivo, permitting prolonged residence in the host. Compositions of the present invention can comprise 100% acetylated polypeptides, but more typically acetylation will range from about 70% to 50%, but may be as low as 30% or less, as desired.

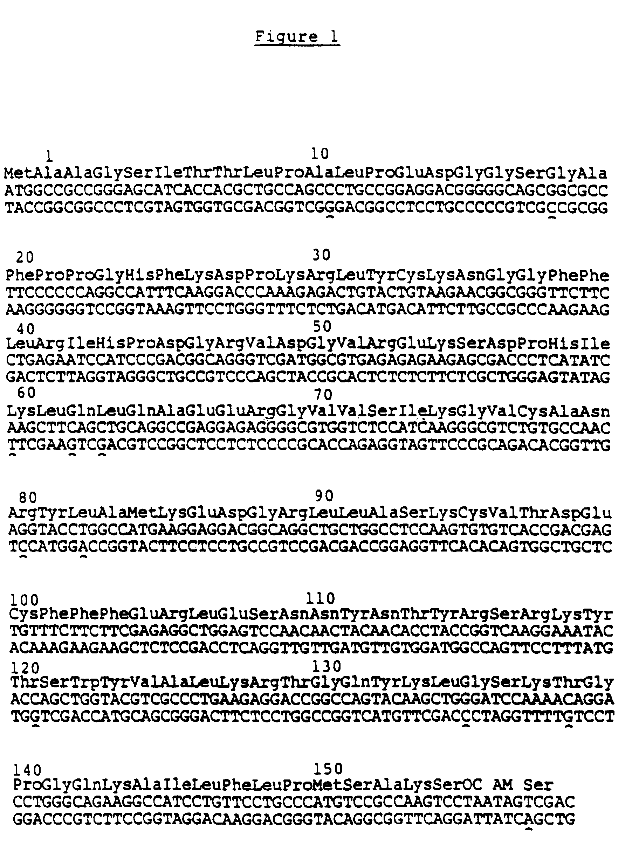

The FGF's of the present invention fall into two general categories, basic FGF and acidic FGF. As used herein, basic FGF's will commonly have a pI between about 9.0 and 10.0, preferably about 9.6, and be capable of binding heparin. Basic FGF is an angiogenic factor and is mitogenic for a wide variety of normal diploid mesoderm-derived and neurocrest-derived cells, including granulocytes, adrenal cortical cells, chrondocytes, myeloblasts, and vascular endothelial cells, and vascular smooth muscle cells (see, United States Patent 5,155,214 the basic application of which was filed December 31, 1987). In accordance with the present invention, basic FGF has been produced in various cleaved forms, but a preferred form is amino-terminal acetylated basic FGF (1-154), with the numbering beginning with the alanine residue immediately following the initiation codon, as shown for human basic FGF in Figure 1 (see, International Patent Application WO87/01728, published March 26, 1987.)

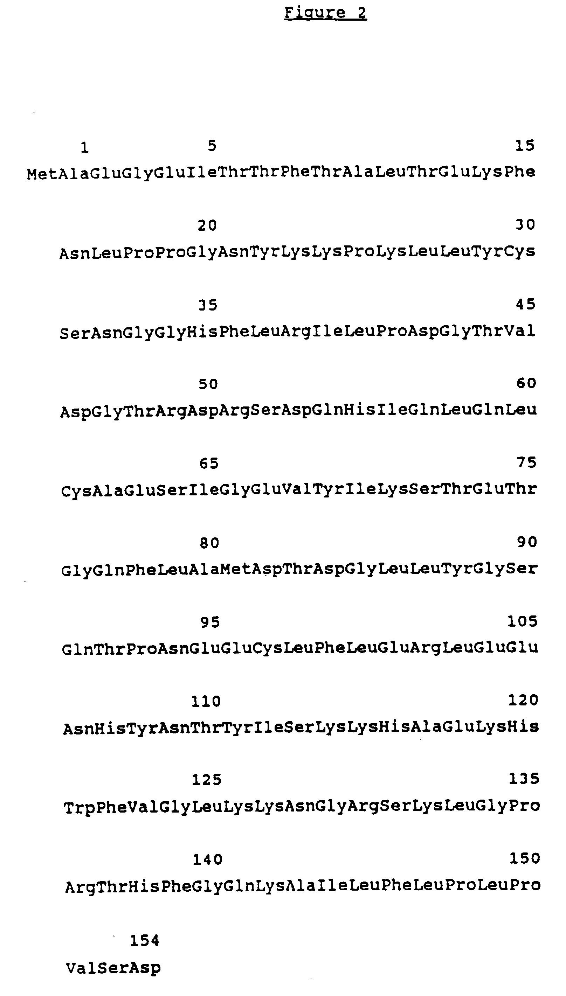

As used herein, acidic FGF refers to a protein that has a pI substantially less than 7.0, typically about 5.0 to about 6.0, and is also capable of binding to heparin. Generally, acidic FGF is active as a mitogen on many of the same types of cells as basic FGF, such as fibroblast, vascular and corneal endothelial cells, chrondocytes, osteoblasts, myeloblasts, smooth muscle cells and glial cells. Also, similar to basic FGF, a preferred acidic FGF is about 154 amino acids when utilizing the same numbering system such, as shown for human basic FGF in Figure 2 (see, European Application No. EP-A-0 259 953 published March 16, 1988.)

Both acidic and basic FGF's are known to exist in various microheterogeneous forms, i.e., the 154 amino acids may be subject particularly to proteolysis, but to additional modifications as well. Thus, additional forms, offered by way of example and not limitation, include human acidic FGF's (9-154), (13-154) and (16-154) and human basic FGF's (9-154) and (10-154). Thus, as used herein, authentically amino-terminally processed FGF's refers to such microheterogeneous forms, some of which will typically be acetylated. Of course, those skilled in the art will recognize that the FGF's of the present invention may also be subject to allelic differences and standard mutations, such as internal deletions, additions or substitutions of many of the amino acid residues, provided one or more of the desired biological activities remain.

To prepare the FGF polypeptides of the present invention in yeast, appropriate DNA sequences encoding the polypeptides must be either isolated or chemically synthesized by assembly of nucleotide bases in accordance with standard techniques. Preferably, chemical synthesis will be utilized, wherein all the nucleotides corresponding to sequences from both strands of the desired gene or synthesized in overlapping sections, for subsequent assemblage into full-length genes.

Once obtained, these genes are inserted into vectors, such as yeast extrachromosomal elements containing a yeast promoter, which is capable of directing internal expression of the desired proteins. These vectors will typically also include an initiation codon, fused immediately upstream from the gene, and a termination codon immediately downstream from the gene. Suitable strong promoters include alcohol dehydrogenase 1 and 2, triosephosphate isomerase, and any other well known promoters. To ensure proper acetylation, however, secretory signal sequences are typically avoided to ensure sufficient acetylation. A common yeast terminator sequence, such as the TPI-1 terminator, may also be utilized. All of these DNA fragments may be ligated in accordance with well known techniques, such as described in Maniatis et al., Molecular Cloning: A Laboratory Manual, Cold Spring Harbor Laboratory 1982.

After preparation of the desired DNA construct, the vector is transformed into the desired yeast host by standard techniques. A preferred host is S. cerevisiae with reduced protease levels, but preferably other than an NAT 1 (N-terminal acetyl transferase) minus mutant. A particularly preferred strain of S. cerevisiae is the one designated JSC302, which has been deposited with the American Type Culture Collection, Rockville, Maryland U.S.A. By overexpression of NAT 1 and ARD 1 (arrest defective), which apparently must be overexpressed together to provide additional acetylase activity, substantially increased acetylated FGF's can be produced. To obtain such overexpression, NAT 1 and ARD 1, under the control of strong promoters, are integrated into separate hosts, which are then mated to form diploids suitable for transformation of the desired expression vector containing FGF.

The transformed yeast cells may be selected by growth on conventional complex medium, which well vary depending upon the particular vector utilized. Once selected, transformants containing the vectors are cloned, high producers selected, and then grown up in the appropriate media.

When the yeast cells have been grown to the desired density, the intracellular FGF's of the present invention may be obtained by first lysing the cells, and then isolating the desired protein by standard purification techniques. Such techniques are well known to those skilled in the- art, and can include affinity chromatography (particularly, heparin based), electrophoresis, dialysis, HPLC or other column chromatography, and the like. The FGF's maybe purified to homogeneity, typically at least 95% pure, and as much as 98% to 99% pure, or more.

The acidic and basic FGF's of the present invention will have substantially the same amino acid sequences as the naturally occurring proteins. As noted previously, microheterogeniety may result in cleavage of varying amino acid stretches from the protein, typically from the amino-terminal end. Five or more forms of an FGF polypeptide may be purified from a single yeast culture, but more typically, two or three forms are produced. Usually. at least about 50% or more of the FGF forms will be acetylated, with the other forms varying from a low of 2% to 5% percent. up to about 40% to 50% of the mixture.

Particularly preferred compositions contain only a single form of FGF. Such a composition may be obtained from microheterogeneous FGF preparations by additional chromotagraphy or electrophoresis steps. Alternatively. human basic FGF (9-154) is expressed in yeast without microheterogeneity.

The substantially pure FGF polypeptides of the present invention can be combined with the pharmaceutically acceptable carrier to form pharmaceutical compositions. which may be administered to patients either intravenously, subcutaneously, intramuscularly, or orally. Required doses will, of course, vary with the particular condition being treated, with the severity of the condition and with the duration of the desired treatment. Suitable concentrations will also vary widely, usually between about 1 and 50 mg/ml, preferably between about 10 to 100 mg/ml. A therapeutically effective amount sufficient to accelerate the rate of appearance and increase of new fibroblasts in vivo will be in the range of 1 to 5 mls of the concentrated preparation. Similar concentrations, but typically lower, may be used to enhance growth or viability of FGF-receptive cells in vivo, such as in cell culture.

These proteins are often administered in the forms of pharmaceutically acceptable nontoxic salts, such as acid addition salts or metal complexes, e.g. zinc, iron or the like. The proteins of the present invention should be administered under the guidance of a physician. If desired, proteins may be administered in conjunction with suitable carriers. diluents in stabilizers, as well as other therapeutic agents, such as other mitogens. including platelet derived growth factor, epidermal growth factor, insulin-like factors. and any of the well known transforming growth factors, such as TGF-a. TGF-, or the like.

The purified recombinant authentic FGF's of the present invention have been shown to be active in a variety of systems. These include standard assays of cell proliferation, such as fibroblasts and endothelial cells, as well as in models of angiogenesis. Thus, these FGF's can be utilized in the wide range of applications in which other FGF's have been demonstrated active. These include in vivo models of nerve regeneration, wound repair, ischemia, and corneal repair. Moreover, as a neutrophic factor capable of promoting neuron survival and differentiation, the present FGF's will permit the development of therapeutic products useful in the central and peripheral nervous systems, such as for treating cell senescence, neuronal regression, and cell death.

The following examples are offered by ways of illustration, not by limitation.

### EXPERIMENTAL

### I. Materials and Methods

The following general materials and methods were utilized to prepare the DNA constructs and expressed FGF proteins of the present invention. Also, the following abbreviations are used:
baFGF refers to bovine acidic FGF, haFGF refers to human acidic FGF, bbFGF refers to bovine basic FGF and hbFGF refers to human basic FGF.

**A. DNA Synthesis and Gene Assembly** Oligonucleotides were synthesized by the phosphoramidite method using Applied Biosystems 380A synthesizers. -Cyanoethoxyphosphoramidite intermediates (American Bionetics, Hayward, California), and o-nitrophenyltetrazole activating agent (American Bionetics) were used for the synthesis of oligonucleotides varying in length between 18 and 45 bases. Typically, a full gene comprised of approximately 22 such oligonucleotides with maximum overlap between complementary oligonucleotides. Purification and phosphorylation of each oligonucleotide has been described in Urdea, M., et al., (1983) Proc. Natl. Acad. Sci. U.S.A. 80:7461-7465. The mixed oligonucleotides heated to 90 and allowed to cool to 25 over 3h in a buffer containing 20mM Tris-HCl, pH 8.0, 10mM MgC12, and 10mM dithiothreitol. After annealing, the mixture was adjusted to 3mM ATP. Ligation for 15min at 25 with T4 DNA ligase (5mL. New England Biolabs. 4X105U/ml) was followed by ethanol precipitation and digestion with Xba-1 and Sal-1. Each synthetic bovine gene was purified by polyacrylamide gel electrophoresis on 7% acrylamide, electroeluted and cloned into Xba-1/Sal-1 digested pHG100. The corresponding human genes were similarly cloned into Nco-/Sal-1 digested pBS100 (Barr, P., et al.. (1987) Vaccine 5:90-101). These plasmids were digested with BamH1 to release expression "cassettes", which were cloned into the BamH1 digested and alkaline phosphatase treated yeast piasmid pAB24 (Fig. 3). Gene sequences and subsequent expression vector junction sequences were verified by M13 dideoxy sequencing. For the several particularly G-C rich regions of the basic FGF genes, the dGTP analog, 7-deaza-dGTP, was used to resolve compressed areas of sequence information (Barr, P., et al., (1986) BioTechniques 4:428432).

**B. Plasmids.** pHg100 is a pBR322 derived plasmid analogous to pAB114 (Brake, A., et al., (1984) Proc. Natl. Acad. Sci. U.S.A. 81:4642-4646). Each plasmid contains S. cerevisiae a-factor promoter, leader and terminator sequences flanking the a-factor structural gene in pAB114, and a synthetic human interleukin-2 (hIL-2) gene in pHG100. In addition, pHG100 was modified by introduction of silent mutations encoding a unique Xba-1 cloning site immediately 5' to the mature hIL-2 coding sequence (Fig. 4). For expression studies, these a-factor leader-synthetic gene constructs were used to the ADH-2/GAPDH promoter (Barr, P., et al., (1987) Vaccine 5:90-101), to give vectors designated pA/Ga-factor bFGF's (Fig. 3[b]). pBS100 contains a BamH1 expression "cassette" consisting of the hybrid ADH-2/GAPDH promoter and the GAPDH transcriptional terminator. These control elements flank a region of the human immunodeficiency virus (HIV) env gene (Barr, P., et al., (1987) Vaccine 5:90-101). Cloning sites for these constructions are Nco-1, which encodes the methionine initiation codon (Fig. 4[c]), and Sal-1, which is situated downstream of termination codons of the gene to be expressed. The yeast plasmid pAB24, described in Barr P., et al., (1986) BioTechnology 5:486-489, contains selectable markers for growth in either uracil or leucine deficient media (Fig. 3). The use of ptac5SOD for expression of hSOD fusion proteins in E. coli is described in Steimer, K., et al., (1986) J. virol. 58:9-16.

**C. Strains.** For transformation and expression in E. coli, strain D1210 was used (Maniatis, T., et al., (1982) Molecular Cloning, A Laboratory Manual, Cold Springs Harbor Laboratory, Cold Spring Harbor, New York). The S. cerevisiae strains utilized are as follows: AB110 (Mata, leu 2-3, 112ura3-52, pep4-3, his4-580, [cir]); 2150-2-3 (Mata leu2, adel, [cir]); AB116 Mata, leu2, trp-1, ura3-52, prB1-1122, pep4-3, prC1-407, [cir]). Yeast transformations were performed as described previously in Hinnen, A., et al., (1978) Proc. Natl. Acad. Sci. U.S.A. 75:1919-1933.

**D. Purification of FGF's from recombinant bacterial and yeast cultures.** Each of the recombinant FGF's was purified by heparin Sepharose chromatography (Shing, Y., et al., (1984) Science 223:1296-1299). Briefly, extracts from lysed bacteria or yeast were loaded directly onto columns of heparin Sepharose (Pharmacia) in Tris-CL buffer 10mM, pH 7.0, 1mM EDTA) and eluted with 0.6M NaC1 (for acidic FGF's) and 1.0M NaCl (for basic FGF's). For E. coli-derived baFGF, chromatography on CM-sephadex (Pharmacia) preceded the heparin column. For elution from heparin Sepharose, NaC1 gradients up to 2.0M (acidic FGF's) and 3.0M (basic FGF's) were used. For final purification of E. coli-derived bbFGF, gel filtration on Ultrogel AcA54 (LKB) in 20mM Tris-CL buffer, (pH7.5, 0.1mM EDTA, 0.3M NaC1) was also included.

The N-terminally acetylated and unblocked forms of hbFGF were separated by reverse phase HPLC on a Vydac C-4 column (0.46 x 25cm). The column was equilibrated with 0.1% trifluoroacetic acid (TFA), and hbFGF's were eluded using a gradient of 32 to 34% acetonitrile. N-terminal sequence analysis of all purified FCF's was performed using an Applied Biosystems 470A gas phase sequenator with on-line HPLC analysis of the PTH-amino acids.

**E. Peptide Mapping.** The two forms of hbFGF resolved by HPLC were reduced with DTT and treated with vinylpyridine to block the cysteine residues. After digestion with Staphylococcus aureus V8 protease, the peptides were separated by HPLC on a Vydac C-18 column (0.46 x 25cm) in 0.1% TFA, using a 50 minute gradient of 10 to 40% acetonitrile. Each peak was collected and subsequently identified by amino acid composition analysis.

**F. Mass Spectrometry.** Protein (1-2 nmol) was dissolved in ammonium bicarbonate (150ml, 50mM, pH 7.8, 0.1mM in (Ca2+) and trypsin (1% by weight) was added. Samples were incubated at room temperature. After 1h a further 1% by weight of trypsin was added, and the digestion was continued for one more hour. Digests were terminated by freezing and lyophilization.

Assignments of protonated molecular ions of N-terminal peptides was established using a Kratos MS50S double-focusing instrument equipped with a high-field magnet, LSIMS source and a post-accelerator detector (10KeV) in the positive ion mode (Aberth, W., et al., (1982) Anal. Chem. 54:2029-2034). Samples were applied to the probe tip and dried in vacuo. A matrix of thioglycerol: glycerol (1:1) containing hydrochloric acid (0.1m) was applied prior to insertion into the source. Spectra were scanned from m/z 3000-300.

**G. Mitogen Assay for FGF.** The mitogenic activity of the various FGF's was assayed using density-arrested human foreskin fibroblasts (HFF), vascular endothelial cells and capillary endothelial cells. HFF cells were plated (1 x 104/well) in 96 well microtiter plates in Dulbecco's Modified Eagles Medium (DMEM) with 5% fetal bovine serum (Hyclone, Ogden Utah). After 5 days, dilutions of various FGF preparations were added in 10ml of serum-free DMEM. About 18 hours later, 3H-Thymidine (1mCi/well; Amersham, 25Ci/mmol, 103 mCi/mg), was added. Plates were then incubated for 24h and then the cultures washed with phosphate buffered saline (PBS). Trichloroacetic acid (5%) was added to the wells for 15 minutes followed by methanol for 15 minutes. The plates were then flooded with methanol, and air dried. The contents of each well were then solubilized in 50ml 0.3N NaOH and transferred to vials containing scintillation fluid for counting. Each dilution of FGF sample was assayed in triplicate. One unit of activity is the amount of FGF required to stimulate half-maximal 3H-thymidine incorporation. The specific activity of various preparation was determined by dividing the reciprocal of the dilution yielding half-maximal incorporation by the protein concentration.

The biological activities of the FGF's were also assessed in models that monitor differentiated function. These included neurite outgrowth by PC12 cell (Gospondarowicz, D., et al., (1986) J. Cell Physiol. 127:121-136), prolactin release by GH3 cells (Gospodarowicz, D., et al., (1982) J. Biol. Chem. 257:1266-12278) and aromatase activity in fibroblasts and granulosa cells (Gospodarowicz, D. (1984) Methods for Preparation of Media, Supplements and Substrata for Serum Free Animal Cell Culture, pp. 275-293, Alan R. Liss, Inc., New York). The angiogenic activity of the growth factors was tested in the chick chorioallantoic membrane (Gospodarowicz, D., et al., (1984) Proc. Natl. Acad. Sci. U.S.A. 81:6963-6967), the rat brain (Gimbrone, M., et al., (1974) J. Natl. Cancer Inst. 52:413-427) and kidney capsule (Risua, W. (1986) Proc. Natl. Acad. Sci. U.S.A. 83:3855-3859).

### II. Examples

The 435 bp and 456 bp genes for baFGF (1-140) (numbered in accordance with Gimenez-Gallego, G., et al., (1985) Science 230:1385-1388) and bbFGF (1-146) (numbered in accordance with Esch, F., et al., (1985) Proc. Natl. Acad. Sci. U.S.A. 82:6507-6511), respectively, were synthesized. The constructs incorporated unique restriction enzyme targets for Hind-III and Nar-1 close to the 5'-end of the baFGF and bbFGF genes, respectively. This allowed the facile transfer of each gene into the desired expression systems, using synthetic adapters. The haFGF precursor gene (Jaye, M., et al., (1986) Science 233:541-545) was similarly synthesized and cloned using oligonucleotides of 29 to 44 bases in length. Since mature bovine and human basic FGF's differ in only two amino acids, only seven new molecules of between 18 and 43 bases were syntesized for the synthetic hbFGF precursor gene. These were ligated, along with the appropriate, previously synthesized oligonucleotide sequences, to give the hbFGF gene (Abraham, J., et al., (1986) EMBO J. 5:2523-2528).

### EXAMPLE 1

Bovine FGF Gene Expression. The synthetic genes for bbFGF (1-146) and baFGF (1-140) were expressed in E. coli using a two cistron message driven by the tac-1 promoter (Hallewell, R., et al., (1986) Nucleic Acids Res. 13:2017-2033) (Fig. 3[a]). It has been shown previously that the use of two cistron messages can overcome translation initiation problems that have been attributed to the formation of stem-loop structures around the ribosome binding site (Schoner, B., et al., (1984) Proc. Natl. Acad. Sci. U.S.A. 81:5403-5407). The highly expressed human superoxide dismutase (hSOD) gene to provided the first cistron of the message. This gene was followed by a new ribosome binding site, a stop codon, and an initiation codon for each of the bovine FGF genes (Fig. 4). IPTG induction of E. coli strain D1210 cells transformed with these plasmids allowed the moderate level expression of bovine FGF's).

These genes were also expressed as fusions with the yeast a-factor mating pheromone leader sequence (Brake, A., et al., (1984) Proc. Natl. Acad. Sci. U.S.A. 81:4642-4646). Synthetic adapters were used to fuse each gene to sequences encoding this secretion and processing signal (Fig. 4). This hybrid gene was flanked by the glucose regulatable alcohol dehydrogenase-2 (ADH-2)/glyceraldehyde-3-phosphate dehydrogenase (GAPDH) hybrid promoter described in Cousens L., et al., (1987) Gene(Amst.) 61:265-275 and Barr P., et al., (1987) J. Exp. Med. 165:1160-1171, and the a-factor transcriptional terminator (Brake, A., et al., (1984) Proc. Natl. Acad. Sci. U.S.A. 81:4642-4646). In each case, secreted FGF's were detected in the yeast supernatants by SDS-PAGE analysis of precipitated protein from TCA treated media and by bioassay.

In order to accurately define N-terminal amino acid structures and specific activities of each recombinant bovine FGF, each protein, was purified from both E. coli cells and S. cerevisiae culture media. This was accomplished by heparin-Sepharose chromatography. Using the procedure, bovine FGF samples of greater than 98% purity were obtained. N-terminal amino acid sequence analysis was performed on each of these purified samples, and the results are shown in Table 1.

A homogeneous N-terminus was only observed in baFGF(1-140) expressed in E. coli. However, even this polypeptide did not represent an authentic baFGF(1-140) molecule, in that, as reported previously for baFGF expressed in E. coli (Linemeyer, D., et al., (1987) Biotechnology 5:960-965), the initiation codon derived N-terminal methionine was not removed in vivo. The E. coli derived bbFGF(1-146), although quantitatively processed with regard to methionine removal, was further degraded to a heterogeneous mixture of bbFGF species (Table 1). Similarly, both bovine FGF's were shown to have heterogeneous N-termini when secreted and purified from yeast. In addition to the natural cleavage at the paired basic amino acid processing site, mediated by the kex-2 gene product (Julius, D., et al., (1984) Cell 37:1075-1089), baFGF was further degraded at the N-terminus (Table 1). Also, secreted bbFGF was found to contain a major species which included the processing site-derived arginine in addition to authentic bbFGF(1-146) (Table 1). We ascribe this unusual processing to an inability of kex-2 protease to efficiently mediate the required cleavage between arginine and proline. The cleavage between lysine and arginine observed is most probably mediated by a yeast protease other than kex-2.

### EXAMPLE 2

Human FGF precursor expression. Because of the N-terminal heterogeneity of the recombinant bovine FGF's, the corresponding human FGF's were expressed as precursor forms using an intracellular yeast expression system. Using initiation codons predicted from protein analysis (Story, M., et al., (1987) Biochem. Biophys. Res. Commun. 142:702-709) and DNA sequence data for haFGF (ECGF) (Jaye, M., et al., (1986) Science 233:541-545) and hbFGF (Abraham, J., et al., (1986) EMBO J. 5:2523-2528) respectively, the synthetic precursor genes were fused directly to the ADH-2/GAPDH hybrid promoter (Fig. 4[c]). Plasmids containing these sequences (Fig. 3[c]) were used to transform yeast under conditions of leucine selection. Induction of FGF gene expression occurred concomitantly with depletion of glucose in the yeast media during culture growth (Cousens L., et al., (1987) Gene(Amst.) 61:265-275). Cells were harvested and analyzed for FGF expression as above. Since FGF's are cell associated in mammalian in vitro models, supernatants of these cultures were examined for exported FGF. In each case, we found FGF polypeptides only in the soluble fraction of the disrupted yeast cells. As with mammalian systems, culture media contained very little FGF (less than about 5% of total).

### EXAMPLE 3

Sequence analysis of human FGF's expressed in yeast. The soluble haFGF and hbFGF polypeptides were readily purified using heparin-Sepharose. The effects of endogenous yeast vacuolar proteases on haFGF expression were studied using the pep4-3 wild type strain 2150. A clear difference in gel electrophoretic mobility between haFGF expressed in AB116 versus that from 2150 was found. Accordingly, each purified hFGF was subjected to N-terminal sequence analysis and mass spectrometry. The haFGF precursor was found to be quantitatively blocked at the N-terminus, as was LSOD (Hallewell, R., et al., (1987) Biotechnology 5:363-366). Mass spectral analysis of tryptic fragments of this polypeptide showed a protonated molecular ion corresponding to the acetylated N-terminal peptide Ac-AEGEITTFRALTEK at m/e 1552. No peptide corresponding to m/e 1510 (unblocked N-terminus) was observed. Thus, as with natural hSOD, yeast derived recombinant hSOD (Hallewell, R., et al., (1987) Biotechnology 5:363-366), natural haFGF (ECGF) (Burgess, W., et al., (1986) Proc. Natl. Acad. Sci. U.S.A. 83:7216-7220) and baFGF (prostatropin) (Crabb, J., et al., (1986) Biochemistry 25:4988-4993), the blocking group was shown to be the acetyl moiety. This data, together with amino acid sequence analysis, also showed that endogenous yeast methionyl aminopeptidase was able to efficiently cleave the initiation codon-derived methionine residue prior to acetylation.

The haFGF purified from yeast strain 2150, was shown to be a mixture of three major species cleaved after residues Phe8 (26%), Thrl2 (11%), Phel5 (7%) together with the blocked precursor (54%). This observation underscores the susceptibility of N-terminal amino acid sequences of FGF's to proteolysis by aspartyl proteases, such as reported in bbFGF (Klagsbrun, M., et al., (1987) Proc. Natl. Acad. Sci. U.S.A. 84:1839-1842).

Purification and analysis of the expressed human basic FGF precursors showed that the N-terminal methionine was quantitatively removed. Surprisingly, however, despite the homology between the FGF's in their N-termini, experiments in peptide mapping, quantitative amino acid sequence analysis and mass spectrometry showed that hbFGF was only partially modified by acetylation. Thus, reverse phase HPLC of heparin sepharose-purified hbFGF resulted in the isolation of two distinct forms of the polypeptide in approximately equimolar proportions. Isolation and identification of V8 protease-derived peptides showed that the difference occurred in the N-terminal peptide. When tryptic digests of the hbFGF mixture were analyzed by mass spectrometry, a protonated molecular ion corresponding to the acetylated N-terminal peptide Ac-AAGSITTLPALPEDGGSGAFPPGHFK was observed at m/e 2537. In addition, the unblocked species was observed at m/e 2495. In agreement with the HPLC data, relative peak heights indicated that the two species were present in roughly equimolar quantities. This situation in some way mimics the results of isolation of basic FGF's from mammalian tissue in that hbFGF from human benign prostatic hyperplastic tissue was found to contain the unblocked N-terminus (Story, M., et al., (1987) Biochem. Biophys. Res. Commun. 142:702-709). Material isolated and purified from a human hepatoma was, however, found to be blocked (Klagsbrun, M., et al., (1987) Proc. Natl. Acad. Sci. U.S.A. 84:1839-1842), as was a similar higher molecular weight bbFGF from bovine pituitary (Ueno, N., et al., (1986) Biochem. Biophys. Res. Commun. 138:580-588).

### EXAMPLE 4

Biological activities of recombinant FGF's. In order to quantitatively assess the specific activity of each polypeptide or mixture of related polypeptides, their mitogenic activity for human foreskin fibroblasts was assayed. The specific activities of the various preparations are shown in Table 1. The capacity of the recombinant proteins to stimulate endothelial cell proliferation is indistinguishable from that of the native mitogens. The recombinant hbFGF was also tested and shown to be active in assays of angiogenesis, neurite outgrowth, neuronal survival in vitro and on the regulation of differentiated function of granulosa cells and fibroblasts.

### EXAMPLE 5

Construction of modified ("short") rhbFGF. Human basic FGF's (9-154) and (10-154) can be prepared by modifying the N-terminus of the plasmid pAB24 A/G-hbFGF to remove the initial eight and nine amino acids, respectively. The plasmid was treated to excise the Nco-1 to Nar-1 fragment, and the following synthetic oligonucleotides were inserted, each having the Nco-1 and Nar-1 overhangs: 
hbFGF (9-154)
MetProÅlaLeuProGluAspGlyGlySerGly
CATGCCAGCCCTGCCGGAGGACGGGGGCAGCG
GGTCGGGACGGCCTCCTGCCCCCGTCGCCGC
 
hbFGF (10-154)
MetAlaLeuProGluAspGlyGlySerGly
CATGGCCCTGCCGGAGGACGGGGGCAGCGG
CGGGACGGCCTCCTGCCCCCGTCGCCGC
 These plasmids are capable of expressing the desired FGF forms in yeast, with hbFGF (10-154) exhibiting partial acetylation, and the genes can be expressed using the ADH2/GAPDH promoter, as described previously (Barr, et al., J. Biol Chem., 263:16471-16478, 1988).

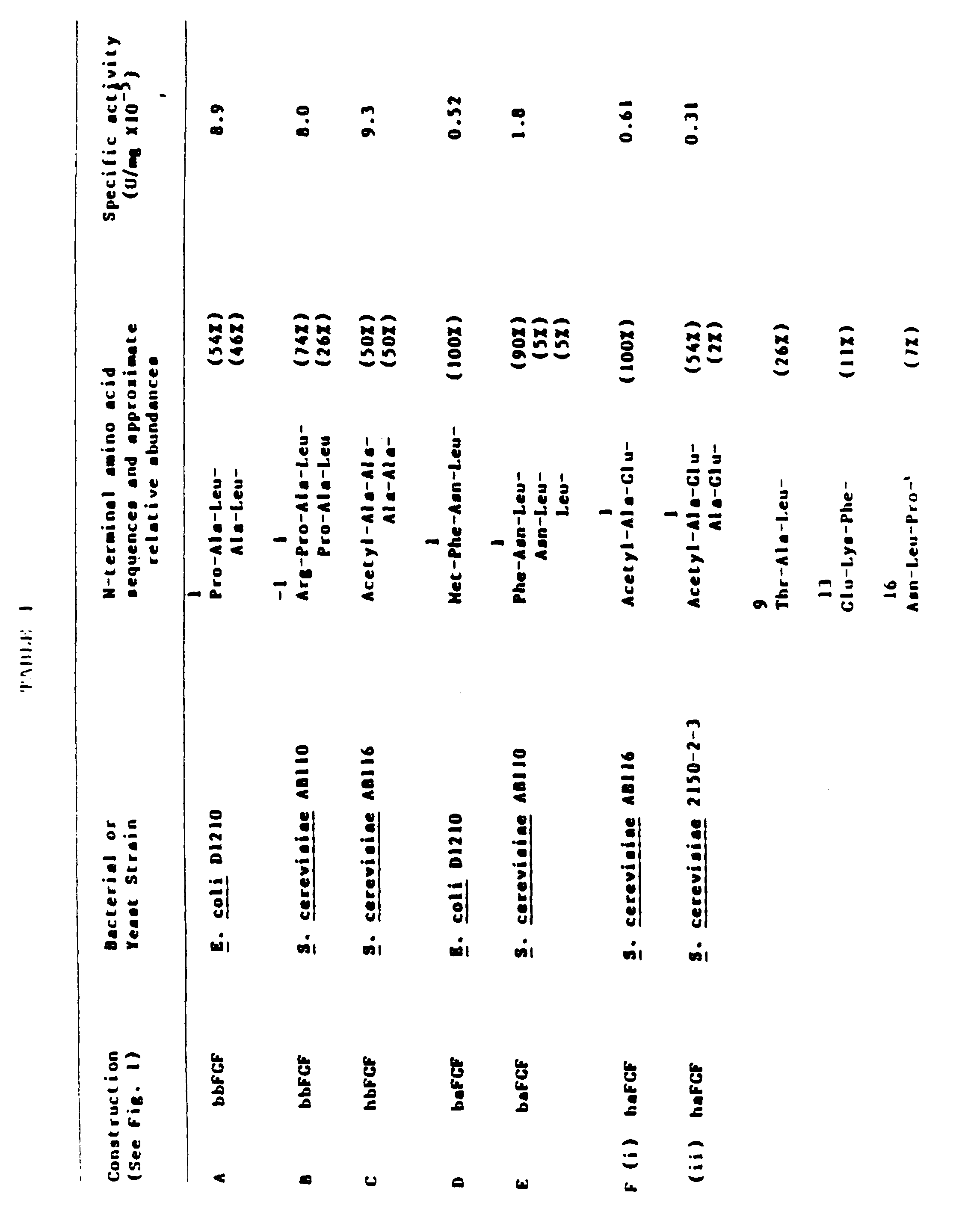

### EXAMPLE 6

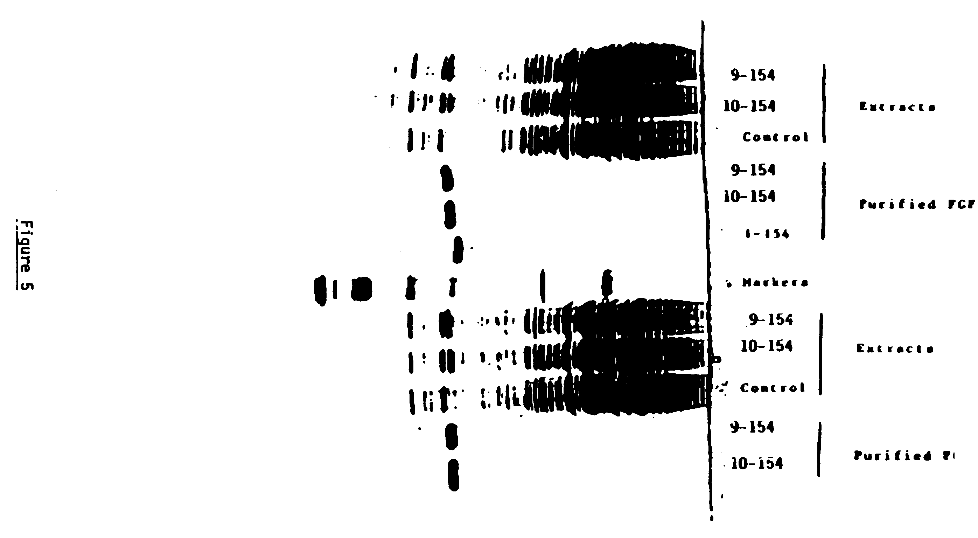

Expression and characterization of the "short" rhbFGF's. These rhbFGF's were expressed internally in yeast as taught in Example 5 and purified starting with cell breakage and centrifugation. As shown in Figure 5, rhbFGF was visible on a gel of the clarified yeast extracts, but absent from cell extracts containing a control plasmid. These proteins were purified by Heparin-5PW HPLC chromatography, which yielded nearly homogeneous material, Figure 5. When more rhbFGF (about 15 ug) was electrophoresed to detect any minor bands, two very minor, high molecular weight contaminants were evident, but no equivalent of 19.5 kDa rhbFGF was seen. These short rhbFGF's bound normally to heparin, indicating that these short rhbFGF's are active. The expression levels were higher in the 9-154 form than in the 10-154 form. For both forms, JSC302 is the preferred yeast host, as for 1-154 rhbFGF. The expression of rhbFGF 9-154 in JSC302 was estimated to be 24 mg per liter of yeast culture, which is in the same range of expression as rhbFGF 1-154 (about 35 mg/liter).

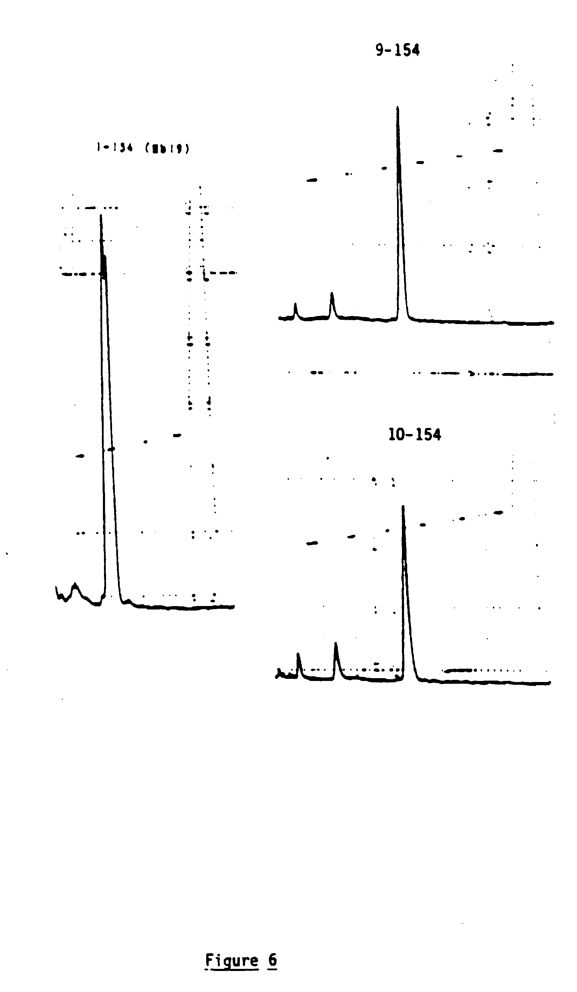

The purified rhbFGF 9-154 and rhbFGF 10-154 form JSC302 cells were analyzed by C-4 reverse phase chromatography. As seen in Figure 6, both proteins eluted as a single major peak, and at approximately the same percentage of acetonitrile as rhbFGF 1-154. Two small early peaks are also evident, which are almost certainly disulfide-bonded forms of rhbFGF. Most significant, however, is that the main rhbFGF peak is a sharp peak, and not a doublet like the full length 1-154 rhbFGF. Amino terminal sequencing of the first 22 residues of the 9-154 form shows the expected sequence of "PALPEDGGSGAFPPGHFKDAPKR". No minor sequences were detected. Thus, it appears that the 9-154 form has a homogeneous N-terminus, and the sharp HPLC peak suggests that there may be no significant microheterogeneity. The 9-154 rhbFGF has an N-terminal proline which is not expected to be acetylated, while the 10-154 form begins with alanine, a preferred residue for acetylation.

### EXAMPLE 7

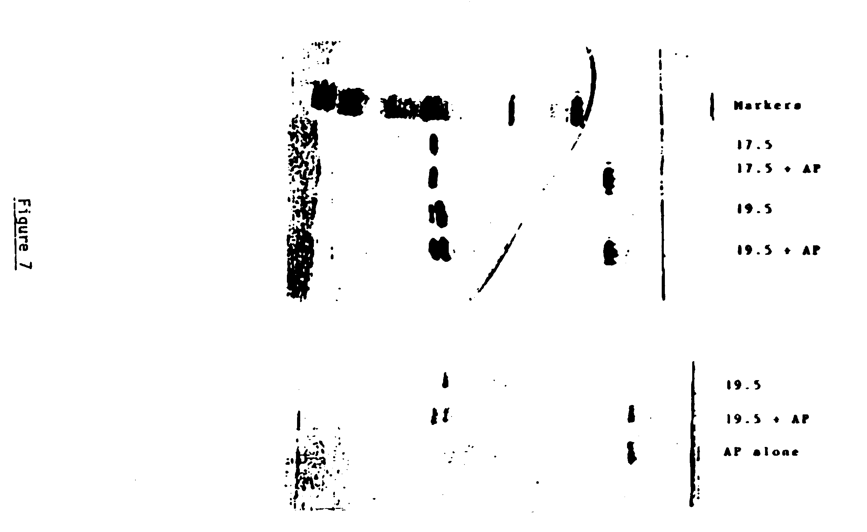

Identification of the 19.5 kDa rhbFGF. The 19.5 kDa form of rhbFGF is a minor species that migrates at a slower rate than the major rhbFGF 17.5 kDa form. Samples of gel-purified 17.5 kDa and 19.5 kDa rhbFGF were incubated with calf alkaline phosphatase to test directly whether phosphorylation causes the reduced mobility of 19.5 kDa rhbFGF. As seen in Figure 7, this treatment had no effect on the 17.5 kDa, but shifted a portion of 19.5 kDa to 17.5 kDa form. On the right hand side of Figure 7, electrophoresis of samples of 19.5 kDa after the alkaline phosphatase treatment shows that a high proportion of the 19,5 kDa converted to 17.5 kDa. (Since phosphoproteins are actually poor substrates for alkaline phosphatase, it is not surprising that all of the 19.5 kDa rhbFGF was not converted to the 17.5 kDa form). This experiment shows that phosphorylation is the cause of the aberrant electrophoretic mobility of 19.5 kDa rhbFGF.

### EXAMPLE 8

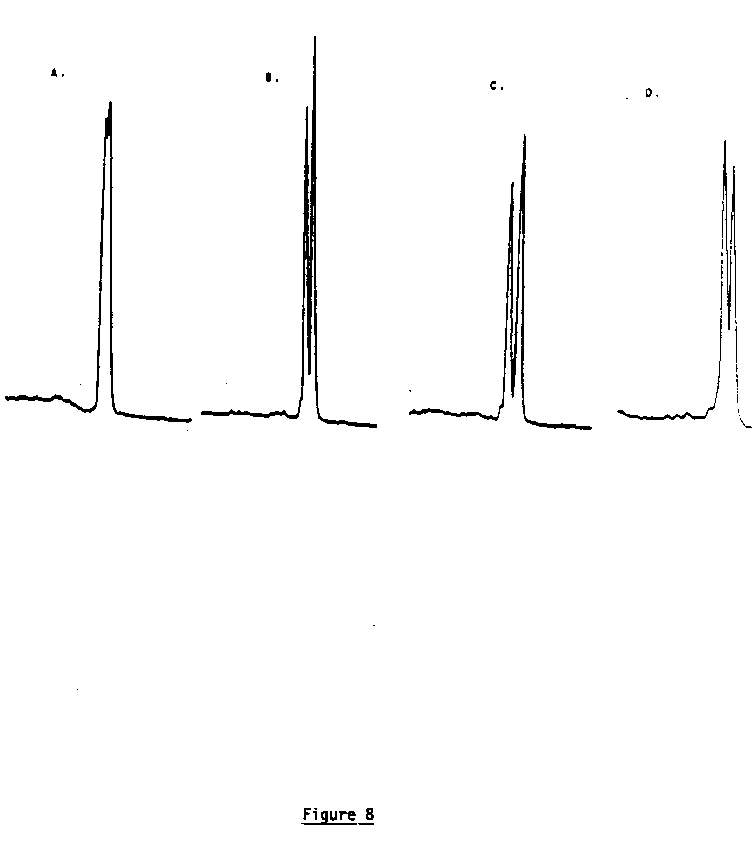

Improved separation of acetylated from unacetylated rhbFGF. Although reverse phase HPLC on a TFA/acetonitrile on a Vydac C-4 column with a very shallow gradient is capable of resolving the two forms of rhbFGF, the separation at room temperature is poor and does not approach baseline resolution (Figure 8A). As shown in Figure 8B and C, simply using the TFA/acetonitrile buffers and the Vydac C-4 column at 50°C resulted in near baseline separation of the two rhbFGF's. Unfortunately, the Vydac C-4 column was unable to withstand the elevated temperatures for longer than one day.

Polymer-based reverse phase columns, Polymer PLRP-S, 0.46 x 25 cm, 300 Angstrom pore size were tested in this system. As seen in Figure 8D, the separation of the two rhbFGF's on the PLRP-S column at 50°C was much improved over the Vydac C-4 at room temperature, and the polymer-based column was better able to withstand high temperature. Resolution of acetylated and unacetylated rhbFGF using a polymeric reverse phase column at 50°C should be applicable to separation of microheterogeneous short forms of rhbFGF.

## Claims

1. A composition comprising a recombinantly produced amino-terminus acetylated human basic fibroblast growth factor (FGF) having the amino acid sequence depicted at positions 1-154 or 10-154 of Figure 1.

2. A composition according to claim 1, further comprising non-acetylated human basic FGF.

3. A composition according to claim 1 or 2, wherein acetylated FGF comprises about 50% of the total FGF.

4. A composition according to any of claims 1 to 3, wherein the specific activity of FGF is at least about 9.3 x 10⁻⁵ units/mg.

5. A composition comprising a recombinantly produced amino-terminus acetylated human acidic fibroblast growth factor (FGF) having an amino acid sequence as depicted at positions 1-154 of Figure 2.

6. A composition according to claim 5, further comprising non-acetylated human acidic FGF.

7. A composition according to claim 5 or 6, wherein acetylated FGF comprises about 50% of the total FGF.

8. A composition according to any of claims 5 to 7, further comprising at least one human acidic FGF selected from the group of FGFs consisting of FGFs (9-154), (13-154) and (16-154) having amino acid sequences depicted at positions 9-154, 13-154 and 16-154, respectively, of Figure 2.

9. A composition according to any of claims 5 to 8, wherein the specific activity of the human acidic FGF polypeptides is at least about 0.61 x 10⁻⁵ units/mg.

10. A method of producing a human fibroblast growth factor (FGF), wherein the FGF is amino-terminus acetylated, said method comprising the steps of:
a) transforming yeast with an expression plasmid comprising a DNA molecule encoding a human FGF, wherein the molecule is under transcriptional control of a promoter functional in yeast;
b) culturing the transformed yeast under conditions suitable for expression of FGF, wherein the FGF is produced intracellularly; and
c) separating FGF from the transformed yeast cells.

11. A method according to claim 10, wherein at least 30% of the FGF polypeptides are amino-terminus acetylated.

12. A method according to claim 11, wherein at least 50% of the FGF polypeptides are amino-terminus acetylated.

13. A method according to claim 11, wherein substantially all of the FGF polypeptides are amino-terminus acetylated.

14. A method according to any of claims 10 to 13, wherein the FGF is basic FGF.

15. A method according to claim 14, wherein the FGF comprises an amino acid sequence as represented by amino acid residues 1-154 of Figure 1.

16. A method according to any of claims 10 to 13, wherein the FGF is acidic FGF.

17. A method according to claim 16, wherein the FGF comprises an amino acid sequence as represented by amino acid residues 1-154 of Figure 2.

18. A method according to any of claims 10 to 17, further comprising the step of purifying the separated FGF with a heparin affinity column.

19. A method according to any of claims 10 to 18, further comprising the step of purifying the separated FGF with an HPLC column.

20. A method according to any of claims 10 to 19, further comprising the step of separating acetylated FGF from non-acetylated FGF.

21. A method according to any of claims 10 to 20, further comprising the step of treating the FGF with phosphatase enzyme.

22. A method according to any of claims 10 to 21, wherein the yeast host cell, before transformation, is derived from *s*. *cerevisiae* strain AB 110, AB 116, 2150, or JSC302.

23. A DNA construct capable of directing the intracellular expression thereof in yeast to yield a processed fibroblast growth factor (FGF), comprising a yeast transcriptional promoter DNA region upstream from an initiation codon, a DNA molecule encoding FGF and a transcription terminator, wherein the promoter is fused at its 3' end to a 5' end of the DNA molecule, and the DNA molecule is followed downstream by the transcription terminator, and wherein the DNA construct is incapable of directing the secretion of the FGF from the yeast.

24. A DNA construct according to claim 23, wherein the promoter is glucose regulatable.

25. A DNA construct according to claim 24, wherein the promoter is a hybrid promoter comprising promoter sequences from alcohol dehydrogenase-2 and glyceraldehyde-3-phosphate dehydrogenase.

26. A DNA construct according to any of claims 23 to 25, wherein the DNA molecule encodes a human FGF comprising an amino acid sequence as represented by amino acid residues 1-154 of Figures 1 or 2.

27. A DNA construct according to any of claims 23 to 25, wherein the DNA molecule encodes a human basic FGF comprising an amino acid sequence as represented by amino acid residues 9-154 or 10-154 of Figure 1.

28. A yeast host cell transformed with the DNA construct of any of claims 23 to 27.

29. A yeast host cell according to claim 28, wherein the cell, before transformation, is derived from *S*. *cerevisiae* strain AB 110, AB 116, 2150, or JSC302.

30. A yeast host cell according to claim 29, wherein the yeast strain is JSC302 (ATCC 20967).

## Patentansprüche

1. Zusammensetzung, umfassend einen rekombinant hergestellten am Aminoende acetylierten menschlichen basischen Fibroblasten-Wachstumsfaktor (FGF) mit der an den Positionen 1-154 oder 10-154 der Figur 1 angegebenen Aminosäuresequenz.

2. Zusammensetzung nach Anspruch 1, weiterhin umfassend einen nicht-acetylierten menschlichen basischen FGF.

3. Zusammensetzung nach Anspruch 1 oder 2, wobei der acetylierte FGF etwa 50 % des gesamten FGF umfaßt.

4. Zusammensetzung nach einem der Ansprüche 1 bis 3, wobei die spezifische Aktivität des FGF mindestens etwa 9,3 x 10⁻⁵ Einheiten/mg ist.

5. Zusammensetzung, umfassend einen rekombinant hergestellten am Aminoende acetylierten menschlichen sauren Fibroblasten-Wachstumsfaktor (FGF) mit einer an den Positionen 1-154 der Figur 2 angegebenen Aminosäuresequenz.

6. Zusammensetzung nach Anspruch 5, weiterhin umfassend einen nicht-acetylierten menschlichen sauren FGF.

7. Zusammensetzung nach Anspruch 5 oder 6, wobei der acetylierte FGF etwa 50 % des gesamten FGF umfaßt.

8. Zusammensetzung nach einem der Ansprüche 5 bis 7, weiterhin umfassend mindestens einen menschlichen sauren FGF aus der Gruppe FGF (9-154), FGF (13-154) und FGF (16-154) mit den an den Positionen 9-154, 13-154 bzw. 16-154 der Figur 2 angegebenen Sequenzen.

9. Zusammensetzung nach einem der Ansprüche 5 bis 8, wobei die spezifische Aktivität der menschlichen sauren FGF-Polypeptide mindestens etwa 0,61 x 10⁻⁵ Einheiten/mg ist.

10. Verfahren zur Produktion eines menschlichen Fibroblasten-Wachstumsfaktors (FGF), dessen Aminoende acetyliert ist, wobei das Verfahren die Schritte umfaßt:
a) Transformation von Hefe mit einem Expressionsplasmid, umfassend ein einen menschlichen FGF codierendes DNA-Molekül, das unter der Transkriptionskontrolle eines in Hefe funktionellen Promotors ist;
b) Züchtung der transformierten Hefe unter für die Expression von FGF geeigneten Bedingungen, wobei der FGF intrazellulär produziert wird; und
c) Abtrennung von FGF aus den transformierten Hefezellen.

11. Verfahren nach Anspruch 10, wobei mindestens 30 % der FGF-Polypeptide am Aminoende acetyliert sind.

12. Verfahren nach Anspruch 11, wobei mindestens 50 % der FGF-Polypeptide am Aminoende acetyliert sind.

13. Verfahren nach Anspruch 11, wobei im wesentlichen alle FGF-Polypeptide am Aminoende acetyliert sind.

14. Verfahren nach einem der Ansprüche 10 bis 13, wobei der FGF basischer FGF ist.

15. Verfahren nach Anspruch 14, wobei der FGF eine Aminosäuresequenz umfaßt, die durch die Aminosäurereste 1-154 der Figur 1 dargestellt ist.

16. Verfahren nach einem der Ansprüche 10 bis 13, wobei der FGF saurer FGF ist.

17. Verfahren nach Anspruch 16, wobei der FGF eine Aminosäuresequenz umfaßt, die durch die Aminosäurereste 1-154 der Figur 2 dargestellt ist.

18. Verfahren nach einem der Ansprüche 10 bis 17, umfassend als weiteren Schritt die Reinigung des abgetrennten FGF mittels einer Heparin-Affinitätssäule.

19. Verfahren nach einem der Ansprüche 10 bis 18, umfassend als weiteren Schritt die Reinigung des abgetrennten FGF mittels einer HPLC-Säule.

20. Verfahren nach einem der Ansprüche 10 bis 19, umfassend als weiteren Schritt die Abtrennung des acetylierten FGF von nicht-acetyliertem FGF.

21. Verfahren nach einem der Ansprüche 10 bis 20, umfassend als weiteren Schritt die Behandlung des FGF mit Phosphataseenzym.

22. Verfahren nach einem der Ansprüche 10 bis 21, wobei die Hefewirtszelle vor der Transformation von dem S. cerevisiae-Stamm AB 110, AB 116, 2150 oder JSC302 stammt.

23. DNA-Konstrukt, das die intrazelluläre Expression davon in Hefe zum Erhalt eines prozessierten Fibroblasten-Wachstumsfaktors (FGF) veranlassen kann, umfassend stromaufwärts eines Initiationscodons einen transkriptionellen Promotor-DNA-Bereich aus Hefe, ein FGF codierendes DNA-Molekül und einen Transkriptionsterminator, wobei der Promotor an seinem 3'-Ende mit einem 5'-Ende des DNA-Moleküls verbunden ist und an das DNA-Molekül stromabwärts der Transkriptionsterminator anschließt, und wobei das DNA-Konstrukt die Sekretion des FGF aus Hefe nicht veranlassen kann.

24. DNA-Konstrukt nach Anspruch 23, wobei der Promotor durch Glucose regulierbar ist.

25. DNA-Konstrukt nach Anspruch 24, wobei der Promotor ein Hybridpromotor ist, umfassend Promotorsequenzen der Alkohol-Dehydrogenase-2 und der Glycerinaldehyd-3-phosphat-Dehydrogenase.

26. DNA-Konstrukt nach einem der Ansprüche 23 bis 25, wobei das DNA-Molekül einen menschlichen FGF codiert, umfassend eine Aminosäuresequenz, die durch die Aminosäurereste 1-154 der Figur 1 oder 2 dargestellt ist.

27. DNA-Konstrukt nach einem der Ansprüche 23 bis 25, wobei das DNA-Molekül einen menschlichen basischen FGF codiert, umfassend eine Aminosäuresequenz, die durch die Aminosäurereste 9-154 oder 10-154 der Figur 1 dargestellt ist.

28. Hefewirtszelle, die mit dem DNA-Konstrukt nach einem der Ansprüche 23 bis 27 transformiert ist.

29. Hefewirtszelle nach Anspruch 28, wobei die Zelle vor der Transformation von dem S. cerevisiae-Stamm AB 110, AB 116, 2150 oder JSC302 stammt.

30. Hefewirtszelle nach Anspruch 29, wobei der Hefestamm JSC302 (ATCC 20967) ist.

## Revendications

1. Composition comprenant un facteur de croissance de fibroblastes humain (FGF) basique, acétylé à l'extrémité amino, produit par recombinaison, possédant la séquence d'acides aminés indiquée aux positions 1-154 ou 10-154 de la Figure 1.

2. Composition selon la revendication 1, comprenant aussi un FGF humain basique non acétylé.

3. Composition selon la revendication 1 ou 2, dans laquelle le FGF acétylé constitue environ 50 % du FGF total.

4. Composition selon l'une quelconque des revendications 1 à 3, dans laquelle l'activité spécifique du FGF est d'au moins environ 9,3 × 10⁻⁵ unités/mg.

5. Composition comprenant un facteur de croissance de fibroblastes humain (FGF) acide, acétylé à l'extrémité amino, produit par recombinaison, possédant la séquence d'acides aminés indiquée aux positions 1-154 de la Figure 2.

6. Composition selon la revendication 5, comprenant aussi un FGF humain acide non acétylé.

7. Composition selon la revendication 5 ou 6, dans laquelle le FGF acétylé constitue environ 50 % du FGF total.

8. Composition selon l'une quelconque des revendications 5 à 7, comprenant aussi au moins un FGF acide humain choisi dans le groupe des FGF constitué par les FGF (9-154), (13-154) et (16-154), possédant les séquences d'acides aminés indiquées respectivement aux positions 9-154, 13-154 et 16-154 de la Figure 2.

9. Composition selon l'une quelconque des revendications 5 à 8, dans laquelle l'activité spécifique des polypeptides FGF acides humains est d'au moins environ 0,61 x 10⁻⁵ unités/mg.

10. Procédé de production d'un facteur de croissance de fibroblastes humain (FGF), dans lequel le FGF est acétylé à l'extrémité amino, ledit procédé comprenant les étapes consistant à:
a) transformer de la levure avec un plasmide d'expression comprenant une molécule d'ADN codant pour un FGF humain, la molécule étant sous le contrôle transcriptionnel d'un promoteur. fonctionnel dans la levure ;
b) cultiver la levure transformée dans des conditions adéquates pour l'expression du FGF, le FGF étant produit par voie intracellulaire ; et
c) séparer le FGF des cellules de levure transformées.

11. Procédé selon la revendication 10, dans lequel au moins 30 % des polypeptides FGF sont acétylés à l'extrémité amino.

12. Procédé selon la revendication 11, dans lequel au moins 50 % des polypeptides FGF sont acétylés à l'extrémité amino.

13. Procédé selon la revendication 11, dans lequel essentiellement tous les polypeptides FGF sont acétylés à l'extrémité amino.

14. Procédé selon l'une quelconque des revendications 10 à 13, dans lequel le FGF est un FGF basique.

15. Procédé selon la revendication 14, dans lequel le FGF comprend une séquence d'acides aminés représentée par les résidus acides aminés 1-154 de la Figure 1.

16. Procédé selon l'une quelconque des revendications 10 à 13, dans lequel le FGF est un FGF acide.

17. Procédé selon la revendication 16, dans lequel le FGF comprend une séquence d'acides aminés représentée par les résidus acides aminés 1-154 de la Figure 2.

18. Procédé selon l'une quelconque des revendications 10 à 17, comprenant aussi l'étape de purification du FGF séparé avec une colonne d'affinité à l'héparine.

19. Procédé selon l'une quelconque des revendications 10 à 18, comprenant aussi l'étape de purification du FGF séparé avec une colonne HPLC.

20. Procédé selon l'une quelconque des revendications 10 à 19, comprenant aussi l'étape qui consiste à séparer le FGF acétylé du FGF non acétylé.

21. Procédé selon l'une quelconque des revendications 10 à 20, comprenant aussi l'étape de traitement du FGF avec une enzyme phosphatase.

22. Procédé selon l'une quelconque des revendications 10 à 21, dans laquelle la cellule hôte de levure, avant transformation, est dérivée de la souche AB 110, AB 116, 2150 ou JCS302 de S. cerevisiae.

23. Produit de synthèse d'ADN capable de diriger son expression intracellulaire dans de la levure pour donner un facteur de croissance de fibroblastes (FGF) transformé, comprenant une région d'ADN promoteur de transcription de levure en amont d'un codon d'initiation, une molécule d'ADN codant pour le FGF et un terminateur de transcription, dans lequel le promoteur est fusionné, à son extrémité 3', avec une extrémité 5' de la molécule d'ADN, et la molécule d'ADN est suivie, en aval, par le terminateur de transcription, et dans lequel le produit de synthèse d'ADN est incapable de diriger la sécrétion du FGF de la levure.

24. Produit de synthèse d'ADN selon la revendication 23, dans lequel le promoteur est un glucose régulable.

25. Produit de synthèse d'ADN selon la revendication 24, dans lequel le promoteur est un promoteur hybride comprenant des séquences promotrices d'alcool déshydrogénase-2 et de glycéraldéhyde-3-phosphate déshydrogénase.

26. Produit de synthèse d'ADN selon l'une quelconque des revendications 23 à 25, dans lequel la molécule d'ADN code pour un FGF humain comprenant une séquence d'acides aminés représentée par les résidus d'acides aminés 1-154 des Figures 1 ou 2.

27. Produit de synthèse d'ADN selon l'une quelconque des revendications 23 à 25, dans lequel la molécule d'ADN code pour un FGF humain basique comprenant une séquence d'acides aminés représentée par les résidus d'acides aminés 9-154 ou 10-154 de la Figure 1.

28. Cellule hôte de levure transformée avec le produit de synthèse d'ADN selon l'une quelconque des revendications 23 à 27.

29. Cellule hôte de levure selon la revendication 28, dans laquelle la cellule, avant transformation, est dérivée de la souche AB 110, AB 116, 2150 ou JCS302 de S. cerevisiae.

30. Cellule hôte de levure selon la revendication 29, dans laquelle la souche de levure est JCS302 (ATCC 20967).
